# FASCICULE DE BREVET EUROPEEN

(11) **EP 2 138 128 B1**
(45) Date de publication et mention de la délivrance du brevet: **14.07.2010**
(21) Numéro de dépôt: 09305535.8
(22) Date de dépôt: 12.06.2009
(51) Int. Cl.: A61F 2/00

(54) **Prothèse herniaire et son procédé de fabrication**
Bruchprothese und ihr Herstellungsverfahren
Hernial prosthesis and method for manufacturing same

(30) Priorité: 27.06.2008 FR 0854306
(43) Date de publication de la demande: 30.12.2009
(73) Titulaire: Aspide Medical, 42350 La Talaudiere (FR)
(72) Inventeur: Dupic, Alexandre, 42350, La Talaudiere (FR); Carteron, Patrick, 42600, Chalain Le Comtal (FR); Wiecek, William, 42160, Bonson (FR)
(74) Mandataire: Dupuis, François

(56) Documents cités:
- WO-A-2004/024030
- WO-A-2006/040760
- FR-A- 2 872 024
- US-A- 5 147 374
- US-A1- 2007 276 484

## Description

L'invention se rattache au secteur technique des prothèses couramment utilisées dans le traitement des hernies ombilicales et des petites éventrations.

Ce type de prothèses est bien connu ainsi que le dispositif introducteur qui lui est associé. Diverses études ont été faites et les publications de brevets en relation sont nombreuses. On cite, par exemple, US 2007/0066980, WO 2004/093690, US 7.101.381, US 2006/0282105, US 5.258.000, US 3.857.395. WO 2004/024030 décrit une prothèse herniaire constituée par une poche composée de deux plaques reliées à un corps comprenant une fourreau de grande hauteur et, en partie supérieure, deux languettes de grande hauteur.

Les prothèses ainsi décrites sont établies sous forme d'une poche présentant un col d'ouverture et d'accès à la partie utile d'un dispositif d'introduction établi sous la forme d'une pince à ailettes assurant un déploiement sous l'action d'une tige de commande pour l'introduction et la mise en forme de la prothèse. Ce dispositif d'introduction de la prothèse est utilisé pour la mise en place de la prothèse in situ, assure le déploiement de la prothèse, puis l'opérateur effectue son retrait.

Les prothèses ainsi décrites présentent un inconvénient important en relation avec la difficile stabilité du dispositif introducteur, à l'intérieur de la poche réceptrice de la prothèse et aussi lors de son enlèvement.

La démarche du demandeur a donc été de réfléchir à une nouvelle configuration de la prothèse herniaire pour répondre à ce problème posé.

Après différentes études et essais, le demandeur a ainsi conçu une nouvelle prothèse herniaire qui est particulièrement bien adaptée pour sa mise en oeuvre par un dispositif d'introduction avec une stabilité accrue de celui-ci par rapport à la prothèse, permettant ainsi un positionnement de la prothèse dans les meilleures conditions.

Selon une première caractéristique, la prothèse herniaire du type comprenant une première partie constituée par une poche définie entre une plaque inférieure en configuration de disque, une plaque de liaison intermédiaire et une plaque supérieure en forme de disque, reliées ensemble par un moyen d'assemblage, la plaque supérieure incluant une ouverture centrale formant col pour le passage et l'introduction, puis le retrait d'un dispositif d'introduction et déploiement optimal de la prothèse in situ, ladite première partie est assemblée à une seconde partie constituant un corps monobloc, et la plaque supérieure comprend, autour de l'ouverture centrale, une pluralité de pattes radiales, et ledit corps comprend trois zones distinctes successives, une première zone médiane sous forme de fourreau de grande hauteur permettant l'introduction et le maintien et guidage pour retrait du dispositif introducteur, ladite partie médiane formant fourreau se prolongeant par le bas par une pluralité de bandes disposées radialement régulièrement et dont les extrémités inférieures sont divisées en deux pour faire apparaître chacune respectivement deux languettes dans une configuration de fixation en Y, chacune de ses languettes, par son extrémité finale, est fixée à l'une des pattes formées sur la poche, et le corps comprend, en partie supérieure, au-delà du fourreau, deux languettes de grande hauteur et qui présentent, en partie supérieure, une découpe longitudinal dans sa largeur pour venir s'accrocher au dispositif introducteur.

Ces caractéristiques et d'autres encore ressortiront bien de la suite de la description.

Pour fixer l'objet de l'invention illustrée de manière non limitative aux figures des dessins où :
- La figure 1 est une vue en perspective de la prothèse, selon l'invention seule.
- La figure 2 est une vue en perspective du dispositif introducteur.
- La figure 3 est une vue éclatée en plan avant assemblage de ses composants et constituant la partie poche de la prothèse.
- La figure 4 est une vue en plan de la partie poche assemblée.
- La figure 5 est une vue en plan de la partie corps de guidage et fixation du dispositif d'introduction et d'attache à la partie poche de la prothèse.
- La figure 6 est une vue illustrant la mise en place du dispositif d'introduction sur la prothèse.

Afin de rendre plus concret l'objet de l'invention, on le décrit maintenant de manière non limitative illustrée aux figures des dessins.

La prothèse herniaire, selon l'invention, est référencée dans son ensemble par (P). Elle comprend essentiellement deux parties assemblées (A - B) réalisées en des matériaux de même nature et, en particulier, le polypropylène. La première partie (A) constitue la poche de la prothèse et comprend une plaque inférieure (1) en configuration de disque, une plaque de liaison (2) intermédiaire annulaire de même configuration et disposée en bordure périphérique de la plaque inférieure, et une plaque supérieure (3) en forme de disque, les trois composants ainsi décrits étant reliés par soudage ou autre moyen d'assemblage en bordure périphérique. La plaque inférieure est pleine et peut présenter, le cas échéant, des ouvertures afin d'assurer le drainage du site d'implantation. La plaque supérieure présente une ouverture centrale (3a) formant col pour le passage et l'introduction du dispositif d'introduction (7). La plaque inférieure est avantageusement en polypropylène non tissé - non tricoté et siliconé afin de limiter les adhérences viscérales, la plaque supérieure est avantageusement en polypropylène tricoté par exemple afin de favoriser l'intégration tissulaire. La plaque de liaison intermédiaire annulaire est avantageusement en polypropylène non tissé, non tricoté.

Dans la mise en oeuvre de l'invention, la plaque supérieure présente, autour de l'ouverture centrale (3a), une découpe circulaire faisant apparaître des pattes (3b) radiales. De préférence, le nombre de pattes est fixé à huit afin de répartir les forces lors du repli ou du déploiement de la prothèse par le dispositif associé. La seconde partie (B) constitue un corps monobloc et a pour fonction d'assurer la mise en place et la stabilité du dispositif introducteur (7) ou liaison avec la poche de la prothèse. Cette seconde partie, constituant le corps, est réalisée en une seule fois et est également en polypropylène non tissé. Elle comprend trois zones distinctes successives, une première zone (4) médiane sous forme de fourreau de grande hauteur permettant l'introduction et le maintien du dispositif introducteur. La partie médiane formant fourreau (4) se prolonge par le bas par une pluralité de bandes (5) et, de préférence quatre bandes disposées radialement régulièrement et dont les extrémités (5a) inférieures sont divisées en deux pour faire apparaître chacune respectivement deux languettes (5b) dans une configuration de fixation en Y. Chacune de ses languettes, par son extrémité finale, est fixée par soudure ou autre à l'une des pattes (3b) formées sur la poche. On a ainsi représenté la disposition de 8 languettes (5b) qui seront écartées en Y après assemblage avec lesdites pattes. Par ailleurs, le corps comprend, en partie supérieure, au-delà du fourreau (4), deux languettes (6) de grande hauteur et qui présentent, en partie supérieure, une découpe (6a) longitudinale dans sa largeur pour venir s'accrocher sur les anneaux de prise (7a) du dispositif introducteur (7). Ces languettes sont ainsi positionnées le long du tube (7b) extérieur du dispositif (7).

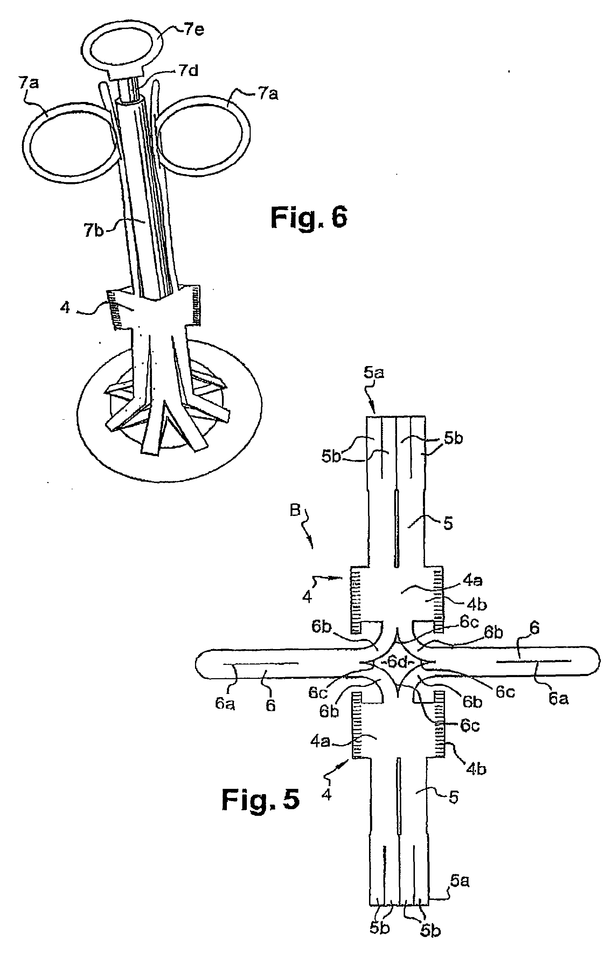

On a, par ailleurs, représenté figure 5, en vue à plat, la configuration du corps lors de sa fabrication. En pratique, le corps de guidage du dispositif introducteur et de liaison avec la poche est réalisé à partir d'une plaque de matériau polypropylène non tissé - non tricoté qui est prédécoupée à plat aux formes à obtenir. La partie fourreau (4) est réalisée aux deux demi-parties (4a-4b) qui seront ensuite assemblées par soudure le long des lignes de jonction (4b) établies à chaque extrémité.

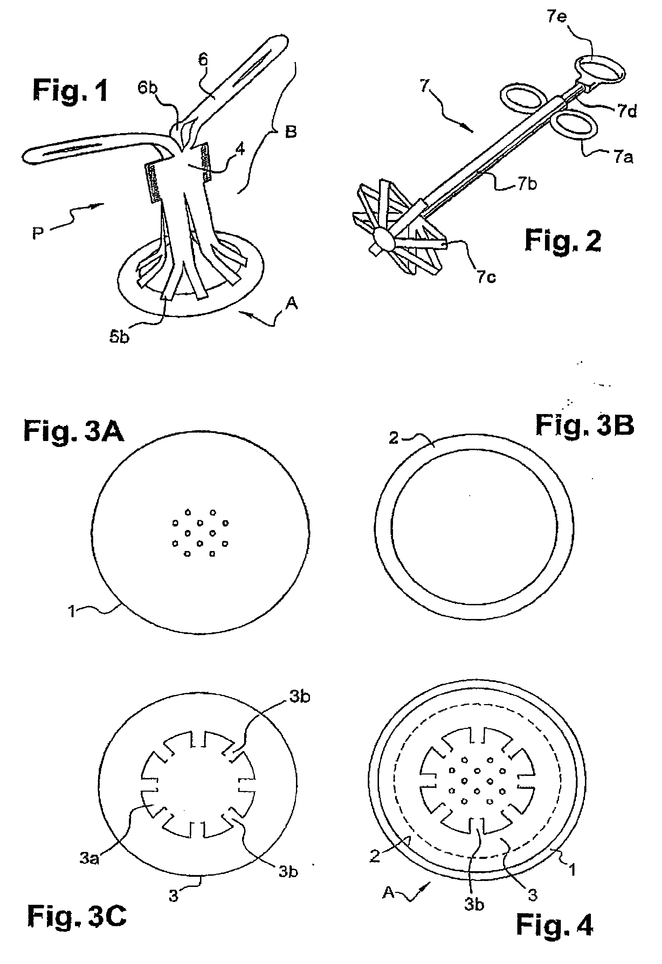

Les bandes et languettes (5 et 5b) sont établies dans le prolongement longitudinal des demi-parties (4a) de fourreau. Les languettes (6) sont par contre établies dans un axe perpendiculaire aux demi-parties (4a) de fourreau pour faire apparaître des pattes de raccordement (6b) coudées. Ainsi, lorsque le corps est assemblé par la liaison des deux parties (4a) du fourreau, les languettes (6) se situent dans le prolongement des lignes de jonction desdites demi parties de fourreau. La base de chaque languette (6) comprend ainsi deux pattes de raccordement (6b) avec une fente (6c) entre elles pour faciliter la tenue desdites languettes. On obtient ainsi une forme en croisillons (6d) typique située au dessus de la partie fourreau. Cette forme en croisillons, outre le fait qu'elle facilite le raccordement des languettes (6) avec le fourreau (4), assure une fonction complémentaire de tenue du dispositif d'introduction. On obtient ainsi deux zones de maintien successives du dispositif (7) en empêchant son basculement. Le guidage du dispositif est donc nettement amélioré. De par sa constitution, le corps est stable verticalement comme il apparaît figure 1 des dessins.

Le dispositif d'introduction (7) comprend un tube (7b) avec, à son extrémité inférieure, une pluralité de languettes (7c) pré pliées susceptibles de définir un parallélépipède déformable en constituant ainsi une pince à ailettes. Une première extrémité desdites languettes est fixée au tube (7b) et l'autre extrémité au bout d'une tige (7d) coulissante dans le tube. Les anneaux de prise (7a) sont réalisés à l'extrémité supérieure du tube (7b). L'extrémité supérieure de la tige (7d) présente un anneau (7e) de prise et de manipulation. Le dispositif (7) est réalisé en polypropylène biocompatible.

Les anneaux (7a) permettent la solidarisation de la prothèse sur le dispositif d'introduction-déploiement pour une introduction aisée de la prothèse.

L'anneau (7e) permet une manipulation facile (par exemple d'une seule main) de l'instrument associé.

Ainsi qu'il apparaît figure 1, la partie corps de la prothèse est de grande hauteur déployé dans son intégralité. La partie fourreau (4) se situe à environ la moitié de la partie corps déployé dans son intégralité et la partie croisillon de guidage complémentaire à environ les 2/3,depuis la poche, de la partie corps déployé dans son intégralité. On a ainsi une meilleure tenue et stabilité du dispositif d'introduction.

## Revendications

1. Prothèse herniaire du type comprenant une première partie (A) constituée par une poche définie entre une plaque inférieure (1) en configuration de disque, une plaque de liaison intermédiaire (2) et une plaque supérieure (3) en forme de disque discale, reliées ensemble par un moyen d'assemblage, la plaque supérieure incluant une ouverture centrale formant col pour le passage et l'introduction puis le retrait d'un dispositif d'introduction de la prothèse in situ, ladite première partie (A) étant assemblée à une seconde partie (B) constituant un corps monobloc,
la plaque supérieure comprenant, autour de l'ouverture centrale (3a), une pluralité de pattes radiales (3b),
ledit corps comprenant trois zones distinctes successives, une première zone (4) médiane sous forme de fourreau de grande hauteur permettant l'introduction et le maintien du dispositif introducteur, ladite partie médiane formant fourreau (4) se prolongeant par le bas par une pluralité de bandes (5) disposées radialement régulièrement et dont les extrémités (5a) inférieures sont divisées en deux pour faire apparaître chacune respectivement deux languettes (5b) dans une configuration de fixation en Y,
chacune de ses languettes, par son extrémité finale, étant fixée à l'une des pattes (3b) formées sur la poche,
le corps comprenant, en partie supérieure, au-delà du fourreau (4), deux languettes (6) de grande hauteur et qui présentent, en partie supérieure, une découpe (6a) longitudinale dans sa largeur pour venir s'accrocher au dispositif introducteur (7).

2. Prothèse herniaire, selon la revendication 1, **caractérisée en ce que** la partie fourreau (4) est réalisée aux deux demi-parties (4a-4b) assemblées le long des lignes de jonction (4b) établies à chaque extrémité, et sert de guidage sur le dispositif d'introduction-déploiement.

3. Prothèse herniaire, selon la revendication 2, **caractérisée en ce que** les bandes et languettes (5 et 5b) sont établies dans le prolongement longitudinal des demi-parties (4a) de fourreau,
et **en ce que** les languettes (6) sont établies dans un axe perpendiculaire aux demi-parties (4a) de fourreau pour faire apparaître des pattes de raccordement (6b) coudées.

4. Prothèse herniaire, selon la revendication 3, **caractérisée en ce que** la base de chaque languette (6) comprend deux pattes de raccordement (6b) avec une fente (6c) entre elles pour faciliter la tenue desdites languettes, en définissant une forme en croisillons (6d) située au dessus de la partie fourreau,
et **en ce que** ladite forme en croisillons a pour fonction de faciliter le raccordement des languettes (6) avec le fourreau (4) et d'assurer une fonction complémentaire de tenue du dispositif d'introduction, en définissant deux zones de maintien du dispositif (7) en empêchant son basculement.

5. Prothèse herniaire, selon la revendication 1, **caractérisée en ce que** la partie corps de la prothèse est de grande hauteur déployé dans son intégralité,
et **en ce que** la partie fourreau (4) se situe à environ la moitié de la partie corps déployé dans son intégralité, et la partie croisillon de guidage complémentaire à environ les 2/3, depuis la poche, de la partie corps déployé dans son intégralité.

6. Procédé de fabrication de la prothèse, selon l'une quelconque des revendications 1 à 5, la mise en oeuvre de la partie corps (B) associée à la partie poche (A) consistant à réaliser, dans une plaque et à plat, prédécoupée, les formes constitutives dudit corps incluant la partie fourreau en deux demi parties (4a - 4b), la partie bande et ses languettes (5 - 5b), et la partie supérieure avec les languettes (6) avec des pattes de raccordement (6b) et fentes (6c) pour la définition d'une forme en croisillon (6d) constitutive avec la partie fourreau des zones de maintien et de guidage du dispositif d'introduction et de mise en forme de la prothèse.

## Claims

1. Hernial prosthesis of the type that includes a first part (A) constituted by a pocket defined between a lower plate (1) in a general disk shape, an intermediate link plate (2) and a disk-shaped upper plate (3), connected together by an assembly means, the upper plate including a central aperture forming a collar for passing through and inserting and then withdrawing a device for inserting the prosthesis in situ, said first part (A) being joined to a second part (B) forming a body cast in one piece,
the upper plate including, around the central aperture (3a), a plurality of radial lugs (3b),
said body including three successive distinct zones, a first median zone (4) in the shape of a sleeve of great height allowing the inserter device to be inserted and held, said sleeve-forming median part (4) extending downwards by a plurality of evenly radially arranged strips (5) whereof the lower ends (5a) are split into two so that each one reveals two tabs (5b) respectively in a Y-shaped fixation layout,
each of its tabs, by its end tip, being secured to one of the lugs (3b) formed on the pocket,
the body including, in the upper part, on the other side of the sleeve (4), two tabs (6) of great height which have, in the upper part, a cut (6a) longitudinal in the width thereof for fastening onto the inserter device (7).

2. Hernial prosthesis, as claimed in claim 1, **characterized in that** the sleeve part (4) is made with the two half-parts (4a-4b) joined along the junction lines (4b) established at each end, and acts as a guide in respect of the insertion-deployment device.

3. Hernial prosthesis, as claimed in claim 2, **characterized in that** the strips and tabs (5 and 5b) are established in the longitudinal extension of the sleeve half-parts (4a),
and **in that** the tabs (6) are established in an axis perpendicular to the sleeve half-parts (4a) to reveal curved coupling lugs (6b).

4. Hernial prosthesis, as claimed in claim 3, **characterized in that** the base of each tab (6) includes two coupling lugs (6b) with a slit (6c) between them to facilitate the holding of said tabs, defining a spider shape (6d) located above the sleeve part,
and **in that** the function of said spider shape is to facilitate the coupling of the tabs (6) with the sleeve (4) and to perform an additional function of holding the insertion device, defining two zones for holding the device (7) preventing it from toppling over.

5. Hernial prosthesis, as claimed in claim 1, **characterized in that** the body part of the prosthesis is of great height when fully deployed,
and **in that** the sleeve part (4) is about half way up the body part when fully deployed, and the additional guidance spider part about 2/3 of the way, from the pocket, up the body part when fully deployed.

6. Method of manufacturing the prosthesis, as claimed in any one of claims 1 to 5, the implementation of the body part (B) associated with the pocket part (A) comprising making, in a plate and flat, pre-cut, the shapes constituting said body including the sleeve part in two half-parts (4a - 4b), the strip part and its tabs (5 - 5b), and the upper part with the tabs (6) with coupling lugs (6b) and slits (6c) for defining a spider shape (6d) that constitutes with the sleeve part zones for holding and guiding the device for inserting and shaping the prosthesis.

## Patentansprüche

1. Hernienprothese mit einem ersten Teil (A), der aus einer vorgegebenen Tasche zwischen einer scheibenartigen unteren Platte (1), einer dazwischenliegenden Verbindungsplatte (2) und einer scheibenförmigen oberen Platte (3), die durch ein Verbindungsmittel miteinander verbunden sind, besteht, wobei die obere Platte eine einen Hals bildende zentrale Öffnung für die Durchführung und Einführung und das anschließende Herausziehen einer Vorrichtung für die Einführung der Prothese in situ umfasst, wobei der erste Teil (A) mit einem zweiten Teil (B), der einen einstückigen Körper bildet, verbunden ist,
wobei der obere Teil um die zentrale Öffnung herum (3a) mehrere radiale Laschen (3b) umfasst,
wobei der besagte Körper drei verschiedene aufeinander folgende Abschnitte besitzt, wovon ein erster mittlerer Abschnitt (4) in Form einer hochgestreckten Hülle die Einführung und den Halt der Einführungsvorrichtung ermöglicht und sich unten durch mehrere Streifen (5) fortsetzt, die regelmäßig radial angeordnet sind und deren untere Enden (5a) halbiert sind, um in Y-förmiger Befestigung jeweils zwei Zungen (5b) hervortreten zu lassen,
wobei jede der Zungen an ihrem äußersten Ende an einer der an der Tasche ausgebildeten Laschen (3b) befestigt ist,
wobei der Körper im oberen Teil über die Hülle (4) hinaus zwei hochgestreckte Zungen (6) umfasst, die am oberen Teil in der Breite einen länglichen Ausschnitt (6a) aufweisen, um sich an der Einführungsvorrichtung (7) einzuhaken.

2. Hernienprothese nach Anspruch 1, **dadurch gekennzeichnet, dass** die Hülle (4) aus zwei Hälften (4a-4b) zusammengesetzt ist, die entlang der an jedem Ende bestehenden Verbindungslinien (4b) miteinander verbunden sind, und als Führung für die Einführungs- und Auffaltungsvorrichtung dient.

3. Hernienprothese nach Anspruch 2, **dadurch gekennzeichnet, dass** die Streifen und Zungen (5 und 5b) in der Längsfortsetzung der Hüllenhälften (4a) ausgebildet sind,
und dass die Zungen (6) in einer senkrechten Achse zu den Hüllenhälften (4a) ausgebildet sind, um abgewinkelte Verbindungslaschen (6b) hervortreten zu lassen.

4. Hernienprothese nach Anspruch 3, **dadurch gekennzeichnet, dass** das Basisteil jeder Zunge (6) zwei Verbindungslaschen (6b) mit einem Schlitz (6c) dazwischen umfasst, um den Halt dieser Zungen zu erleichtern, wobei oberhalb des Hüllenteils (6d) eine Kreuzform beschrieben wird,
und dass diese Kreuzform die Aufgabe hat, die Verbindung der Zungen (6) mit der Hülle (4) zu erleichtern und die Einführungsvorrichtung zusätzlich zu stabilisieren, indem durch zwei Haltezonen der Vorrichtung (7) ihr Umkippen verhindert wird.

5. Hernienprothese nach Anspruch 1, **dadurch gekennzeichnet, dass** der Körperteil der Prothese ganz aufgefaltet eine große Höhe aufweist,
und dass der Hüllenteil (4) sich ungefähr in der Mitte des ganz aufgefalteten Körperteils befindet, während der ergänzende kreuzförmige Führungsteil sich ausgehend von der Tasche auf ungefähr 2/3 des ganz aufgefalteten Körperteils befindet.

6. Herstellungsverfahren der Prothese nach einem der vorhergehenden Ansprüche 1-5, wobei die Fertigung des mit dem Taschenteil (A) verbundenen Körperteils (B) darin besteht, aus einer vorgestanzten und flachen Platte die Bestandteile des besagten Körpers zu fertigen, einschließlich des aus zwei Hälften (4a - 4b) bestehenden Hüllenteils, der Streifen und Zungen (5 - 5b) und des oberen Teils mit den Zungen (6) und deren Verbindungslaschen (6b) und Schlitze (6c) zur Bildung einer Kreuzform (6d) als Bestandteil des Hüllenteils der Halte- und Führungsbereiche der Vorrichtung für die Einführung und Formgebung der Prothese.
